(19) **Europäisches Patentamt / European Patent Office / Office européen des brevets**

(11) **EP 1 969 128 B1**

(12) **EUROPEAN PATENT SPECIFICATION**

(45) Date of publication and mention
of the grant of the patent:
**22.01.2014 Bulletin 2014/04**

(21) Application number: **06849263.6**

(22) Date of filing: **05.12.2006**

(51) Int Cl.:
*A01N 63/02* (2006.01)   *C12P 1/04* (2006.01)

(86) International application number:
**PCT/US2006/046347**

(87) International publication number:
**WO 2007/081455 (19.07.2007 Gazette 2007/29)**

(54) **INDUCTION OF A PHYSIOLOGICAL DISPERSION RESPONSE IN BACTERIAL CELLS IN A BIOFILM**

INDUKTION EINER PHYSIOLOGISCHEN DISPERSIONSANTWORT BEI BAKTERIENZELLEN IN EINEM BIOFILM

INDUCTION D'UNE RÉPONSE DE DISPERSION PHYSIOLOGIQUE DANS DES CELLULES BACTÉRIENNES PRÉSENTES DANS UN BIOFILM

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.12.2005 US 748752 P**

(43) Date of publication of application:
**17.09.2008 Bulletin 2008/38**

(73) Proprietor: **The Research Foundation of State University of**
**New York**
**Albany, NY 12207-2826 (US)**

(72) Inventor: **DAVIES, David, G.**
**Binghamton, NY 13903 (US)**

(74) Representative: **Potter Clarkson LLP**
**The Belgrave Centre**
**Talbot Street**
**Nottingham, NG1 5GG (GB)**

(56) References cited:
**WO-A2-02/27018     US-A1- 2004 235 914**
**US-B1- 6 455 031**

- **DATABASE BIOSIS [Online] BIOSCIENCES INFORMATION SERVICE, PHILADELPHIA, PA, US; 2001, TAGLIENTE D J ET AL: "Development and dispersion of Pseudomonas aeruginosa biofilms" XP009133213 Database accession no. PREV200200233614**

- **DATABASE BIOSIS [Online] BIOSCIENCES INFORMATION SERVICE, PHILADELPHIA, PA, US; 2003, DAVIES D G ET AL: "Autodispersion in Pseudomonas aeruginosa biofilms." XP009133214 Database accession no. PREV200300546547**
- **DATABASE BIOSIS [Online] BIOSCIENCES INFORMATION SERVICE, PHILADELPHIA, PA, US; May 2005 (2005-05), DAVIES D G ET AL: "Autoinduction of Pseudomonas aeruginosa biofilm dispersion" XP009133221 Database accession no. PREV200800237844**
- **IRIE ET AL: "Pseudomonas aeruginosa rhamnolipids disperse Bordetella bronchiseptica biofilms" FEMS MICROBIOLOGY LETTERS, BLACKWELL PUBLISHING, AMSTERDAM, NL LNKD- DOI:10.1016/J.FEMSLE.2005.07.012, vol. 250, no. 2, 15 September 2005 (2005-09-15), pages 237-243, XP005039115 ISSN: 0378-1097**
- **DAVIES DAVID G ET AL: "The involvement of cell-to-cell signals in the development of a bacterial biofilm" SCIENCE (WASHINGTON D C), vol. 280, no. 5361, 10 April 1998 (1998-04-10), pages 295-298, XP002582292 ISSN: 0036-8075**
- **SMITH ET AL.: 'Induction and Inhibition of Pseudomonas aeruginoas Quorum Sensing by Synthetic Autoinducter Analogs' CHEMISTRY & BIOLOGY vol. 10, January 2003, pages 81 - 89, XP026989715**

- ERICKSON ET AL.: 'Pseudomonas aeruginosa Quorum-Sensing Systems May Control Virulence Factor Expression in the Lungs of Patients with Cystic Fibrosis' INFECTION AND IMMUNITY vol. 70, no. 4, April 2002, pages 1783 - 1790, XP008129455
- DAVIES DAVID G ET AL: "A fatty acid messenger is responsible for inducing dispersion in microbial biofilms", JOURNAL OF BACTERIOLOGY, AMERICAN SOCIETY FOR MICROBIOLOGY, WASHINGTON, DC; US, vol. 191, no. 5, 1 March 2009 (2009-03-01) , pages 1393-1403, XP009146308, ISSN: 0021-9193, DOI: 10.1128/JB.01214-08
- K. SAUER ET AL: "Pseudomonas aeruginosa Displays Multiple Phenotypes during Development as a Biofilm", JOURNAL OF BACTERIOLOGY, vol. 184, no. 4, 15 February 2002 (2002-02-15), pages 1140-1154, XP055046161, ISSN: 0021-9193, DOI: 10.1128/jb. 184.4.1140-1154.2002
- Zhang: "Effect of a Pseudomonas Rhamnolipid Biosurfactant on Cell Hydrophobicity and Biodegradation of Octadecane", , 1 January 1994 (1994-01-01), pages 2101-2106, XP055046162, Retrieved from the Internet: URL:http://www.ncbi.nlm.nih.gov/pmc/articl es/PMC201607/pdf/aem00023-0407.pdf [retrieved on 2012-11-30]
- MONTEIRO ET AL: "Molecular and structural characterization of the biosurfactant produced by Pseudomonas aeruginosa DAUPE 614", CHEMISTRY AND PHYSICS OF LIPIDS, LIMERICK, IR, vol. 147, no. 1, 5 May 2007 (2007-05-05), pages 1-13, XP022061222, ISSN: 0009-3084, DOI: 10.1016/J.CHEMPHYSLIP. 2007.02.001
- K. H. MCCLEAN ET AL: "Quorum sensing and Chromobacterium violaceum: exploitation of violacein production and inhibition for the detection of N-acylhomoserine lactones", MICROBIOLOGY, vol. 143, no. 12, 1 December 1997 (1997-12-01), pages 3703-3711, XP055046168, ISSN: 1350-0872, DOI: 10.1099/00221287-143-12-3703
- Mclean: "Evidence of autoinducer activity in naturally occurring biofilms", , 1 January 1997 (1997-01-01), pages 259-263, XP055046174, Retrieved from the Internet: URL:http:// onlinelibrary.wiley.com/store/1 0.1111/j. 1574-6968.1997.tb12653.x/asset/j. 1574-6968.1997.tb12653.x.pdf?v=1&t=ha56axs g&s=5f3c61bd741e1f1507b57b463da28d0a6fc20 f 2d [retrieved on 2012-11-30]
- MILLER M B ET AL: "QUORUM SENSING IN BACTERIA", ANNUAL REVIEW OF MICROBIOLOGY, ANNUAL REVIEWS, US, vol. 55, 1 January 2001 (2001-01-01), pages 165-199, XP009017153, ISSN: 0066-4227, DOI: 10.1146/ANNUREV.MICRO.55.1.165

Remarks:
    The file contains technical information submitted after the application was filed and not included in this specification

## Description

This application claims the benefit of U.S. Provisional Patent Application Serial No. 60/748,752, filed December 9, 2005.

## FIELD OF THE INVENTION

The present invention is directed to a method of inducting a physiological dispersion response in bacterial cells in a biofilm.

## BACKGROUND OF THE INVENTION

Due to the compact nature of biofilm structures, the presumed reduced physiological state of biofilm bacteria and the protection conferred by biofilm matrix polymers, natural and artificial chemical agents are unable to adequately attack and destroy infectious biofilm populations (Costerton et al., "Bacterial Biofilms in Nature and Disease," Annu. Rev. Microbiol. 41:435-464 (1987); Hoiby et al., "The Immune Response to Bacterial Biofilms," In Microbial Biofilms, Lappin-Scott et al., eds., Cambridge: Cambridge University Press (1995)). Increased antibiotic resistance is a general trait associated with biofilm bacteria. When attached, bacteria exhibit a profound resistance, rendering biofilm cells 10 - 1000 fold less susceptible to various antimicrobial agents than the same bacterium grown in planktonic (free floating) culture. For instance, chlorine (as sodium hypochlorite) an oxidizing biocide considered to be one of the most effective antibacterial agents, has been shown to require a 600 fold increase in concentration to kill biofilm cells of *Staphylococcus aureus* when compared to planktonic cells of the same species (Luppens et a/., "Development of a Standard Test to Assess the Resistance of Staphylococcus aureus Biofilm Cells to Disinfectants," Appl Environ Microbiol. 68:4194-200 (2002)). Several hypotheses have been advanced to account for the extraordinary resistance of biofilm bacteria to antibiotics including: (i) reduced metabolic and divisional rates exhibited by biofilm bacteria (particularly those deep within the biofilm); (ii) the biofilm EPS matrix may act as an adsorbent or reactant, reducing the amount of agent available to interact with biofilm cells. Additionally, the biofilm structure may physically reduce the penetration of antimicrobial agents by walling off access to regions of the biofilm; (iii) biofilm cells are physiologically distinct from planktonic bacteria, expressing specific protective factors such as multidrug efflux pumps and stress response regulons (Brown et al., "Resistance of Bacterial Biofilms to Antibiotics: A Growth-Rate Related Effect?" J. Antimicrob. Chemotherapy 22:777-783 (1988); Anwar et al., "Establishment of Aging Biofilms: Possible Mechanism of Bacterial Resistance to Antimicrobial Therapy," Antimicrob. Agents Chemother. 36:1347-1351 (1992); Mah et al., "Mechanisms of biofilm Resistance to Antimicrobial Agents," Trends Microbiol. 9:34-39 (2001); Sauer et al., "Pseudomonas aeruginosa Displays Multiple Phenotypes During Development as a Biofilm," J. Bacteriol. 184:1140-1154 (2002); Stewart, P.S., "Mechanisms of Antibiotic Resistance in Bacterial Biofilms," Int. J. Med. Microbiol. 292:107-113 (2002); Donlan et al., "Biofilms: Survival Mechanisms of Clinically Relevant Microorganisms," Clinical Microbiol. Reviews 15:167-193 (2002); Gilbert et al., "The Physiology and Collective Recalcitrance of Microbial Biofilm Communities," Adv. Microb. Physiol. 46:202-256 (2002); Gilbert et al., "Biofilms In vitro and In vivo: Do Singular Mechanisms Imply Cross-Resistance?" J. Appl. Microbiol. Suppl. 98S-110S (2002)). As detailed molecular studies emerge, it is becoming apparent that each of these factors plays a role in the unusual resistance of biofilms to antimicrobials. Initial treatment is usually effective in killing bacteria only at the margins of biofilm microcolonies. Bacteria deep within these microcolonies are unaffected by the antibiotic and form a nidus for continued dissemination of the infection.

Microbial biofilms in infections and in industrial systems present significant problems due to their recalcitrance to effective treatment.

Detachment is a generalized term used to describe the removal of cells (either individually or in groups) from a biofilm or substratum. Bryers, J.D., "Modeling Biofilm Accumulation," In: Physiology Models in Microbiology. Bazin et al., eds., Boca Raton, FL., Vol. 2, pp. 109-144 (1988) categorized four distinct detachment mechanisms: abrasion, grazing, erosion, and sloughing. These mechanisms have been described principally from the point of view of the chemical and physical environment acting upon biofilm bacteria. Active detachment as a physiologically regulated event has been hinted at by many authors, but few studies have been performed to demonstrate a biological basis for detachment.

One study on the physiological regulation of detachment was carried out by Peyton et al., "Microbial Biofilms and Biofilm Reactors," Bioprocess Technol. 20:187-231 (1995) on *P. aeruginosa.* In their work, it was observed that substrate limitation resulted in a decrease in the detachment rate, presumably a result of reducing the growth rate. Allison et al., "Extracellular Products as Mediators of the Formation and Detachment of Pseudomonas fluorescens Biofilms," FEMS Microbiol. Lett. 167:179-184 (1998) showed that following extended incubation, *P. fluorescens* biofilms experienced detachment, coincident with a reduction in EPS. In *Clostridium thermocellum,* the onset of stationary phase has been correlated with increased detachment from the substratum (Lamed et al., "Contact and Cellulolysis in Clostridium thermocellum via Extensive Surface Organelles," Experientia 42:72-73 (1986)). It has been postulated that starvation may lead to detachment by an unknown mechanism which allows bacteria to search for habitats richer in nutrients (O'Toole et al., "Biofilm Formation as Microbial Development," Ann. Rev. Microbiol. 54:49-79 (2000)).

The transition from a flowing system to a batch culture system has been observed by many labs to result in biofilm detachment. One lab has observed reproducible detachment of biofilm cells of *P. aeruginosa* when flow is arrested in a continuous culture system (Davies, D.G., "Regulation of Matrix Polymer in Biofilm Formation and Dispersion," In Microbial Extracellular Polymeric Substances, pp. 93-112, Wingender et al., eds., Berlin: Springer (1999)).

The release of degradative enzymes has been proposed by others. One such example is found with the gram positive organism *Streptococcus mutans* which 30 produces a surface protein releasing enzyme (SPRE), shown to mediate the release of proteins from the cell envelope (Lee et al., "Detachment of Streptococcus mutans Biofilm Cells by an Endogenous Enzymatic Activity," Infect. Immun. 64:1035-1038 (1996)). Boyd et al., "Role of Alginate Lyase in Cell Detachment of Pseudomonas aeruginosa, " Appl. Environ. Microbiol. 60:2355-2359 (1995) showed that over-expression of alginate lyase causes the degradation of alginate. When a mucoid strain of *P. aeruginosa* was induced to over-express alginate lyase, cells were more easily removed by gentle rinsing from solid medium.

Cell density dependent regulation may also be responsible for the release of enzymes which can degrade biofilm matrix polymers allowing bacteria to disperse from a biofilm. It has been observed at the Center for Biofilm Engineering at Montana State University, USA (Davies, D. G. and Costerton, J. W.) and in the laboratories of Dr. Lapin-Scott at the University of Exeter, UK, that when certain bacteria (including *P. aeruginosa)* reach high cell densities in biofilm cell clusters, the bacteria often undergo a detachment event. Mutants of *P. aeruginosa* which lacked the ability to synthesize the quorum sensing autoinducer $3OC_{12}$-HSL, were susceptible to detachment following treatment with mild detergent (Davies et al., "The Involvement of Cell-to-Cell Signals in the Development of a Bacterial Biofilm," Science 280:295-298 (1998)). Other investigators have demonstrated that homoserine lactones may play a role in detachment. Lynch et al., "Investigation of Quorum Sensing in Aeromonas hydrophila Biofilms Formed on Stainless Steel,: In: Biofilms - The Good, the Bad and the Ugly, Wimpenny et al., eds. Bioline, Cardiff, pp. 209-223 (1999) reported an increase in detachment of *Aeromonas hydrophila* from biofilms and Puckas et al., "A Quorum Sensing system in the Free-Living Photosynthetic Bacterium Rhodobacter sphaeroides," J. Bacteriol. 179:7530-7537 (1997) reported that homoserine lactone production was negatively correlated with cell cluster formation in Rhodobacter sphaeroides.

It has been recognized that *P. aeruginosa* biofilms do not develop into macroscopic biofilm structures in batch culture flasks (at the glass liquid interface). Yet, when medium is pumped continuously through such a flask, (as in a chemostat) a luxurious biofilm develops completely coating the glass surface. When flow is halted in such a system, the biofilm sloughs after a number of days, generally around 72 hrs (Davies et al., "The Involvement of Cell-to-Cell Signals in the Development of a Bacterial Biofilm," Science 280:295-298 (1998)). The inability of biofilms to develop in batch culture has been observed for a number of gram negative and gram positive bacteria as well as mixed cultures of bacteria. This phenomenon demonstrates that there is some aspect of batch growth that is inhibitory to biofilm development.

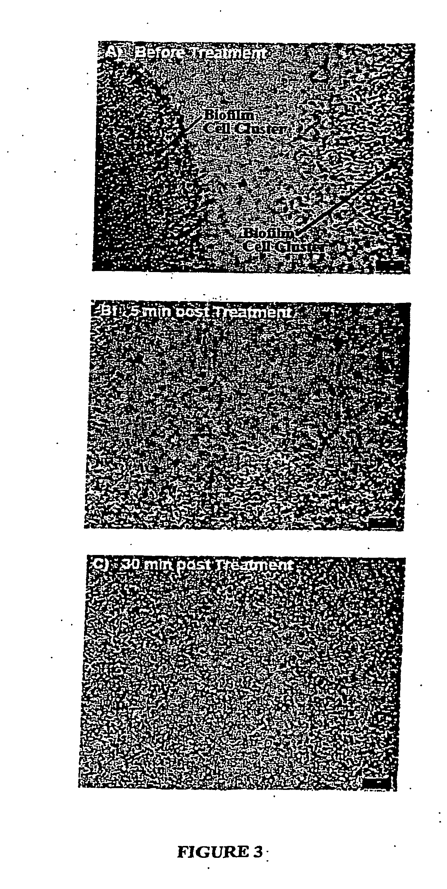

During the last stage of biofilm development, the protein profile of bacteria matches more closely the protein profile of planktonic cells than it does biofilm bacteria from the previous stage, denoted maturation II (see Figure 3 of the current application, and Sauer et al., "Pseudomonas aeruginosa Displays Multiple Phenotypes During Development as a Biofilm," J. Bacteriol. 184:1140-1154 (2002)).

Due to the compact nature of biofilm structures, the presumed reduced physiological state of biofilm bacteria and the protection conferred by biofilm matrix polymers, current natural and artificial chemical agents are unable to adequately attack and destroy infectious biofilm populations (Costerton et al., "Bacterial Biofilms in Nature and Disease," Annu. Rev. Microbiol. 41:435-464 (1987); Hoiby et al., "The Immune Response to Bacterial Biofilms," In Microbial Biofilms, Lappin-Scott et al., eds., Cambridge: Cambridge University Press (1995)).

Davies et al (2003) "Autodispersion in Pseudomonas aeruginosa Biofilms" Biosciences Information Service, Philadelphia, PA, US reports the effects of spent culture medium on dispersion.

Davies *et al* (2005) "Autoinduction of *Pseudomonas aeruginosa* Biofilm dispersion" Biosciences Information Service, Philadelphia, PA, US investigates autoinduction of biofilm dispersion.

The present invention is directed to overcoming these and other deficiencies in the art.

## SUMMARY OF THE INVENTION

We provide a compound which induces a physiological dispersion response in bacterial cells in a biofilm.

We provide a composition which includes the compound and an additive component selected from the group consisting of biocides, surfactants, antibiotics, antiseptics, detergents, chelating agents, and virulence factor inhibitors.

We provide a method of isolating a compound which induces a physiological dispersion response in bacterial cells in a biofilm. The method involves providing a bacterial cell culture and subjecting the bacterial cell culture to a chemical extraction procedure. The chemically extracted bacterial cell culture is subjected to a high pressure liquid chromatography procedure. The method is performed under conditions effective to isolate the compound which induces a physiological dispersion response in bacterial cells in a biofilm.

An aspect of the present invention relates to a method of dispersing a biofilm. The method is set out in claim 1.

A further aspect of the present invention relates to a method of inhibiting formation of a biofilm on a surface as set out in claim 1.

Further aspects of the present invention relate to compositions for use in: treating subjects with bums; treating and/or preventing dental plaque, dental caries, gingival disease, and oral infection; cleaning and/or disinfecting contact lenses; treating and/or preventing acne or other biofilm-associated skin infections on the skin of a subject, and treating and/or preventing a chronic biofilm-associated disease in a subject. The compositions are for administering the compound according to the present invention, under conditions effective to accomplish each respective task.

The present invention addresses the "biofilm problem" by artificially inducing bacteria to undergo biofilm dispersion. The ability to induce dispersion will allow the control of biofilms directly and will improve existing treatments with biocides, topical antibiotics, detergents, etc. The examples of situations in which artificial dispersion would be of benefit include improved cleaning of contact lenses and teeth, improved antiseptic activity in the home, in industry, and in the medical community and enhanced cidal activity for existing antibiotic treatments such as with burn patients infected with *Pseudomonas aeruginosa.*

## BRIEF DESCRIPTION OF THE DRAWINGS

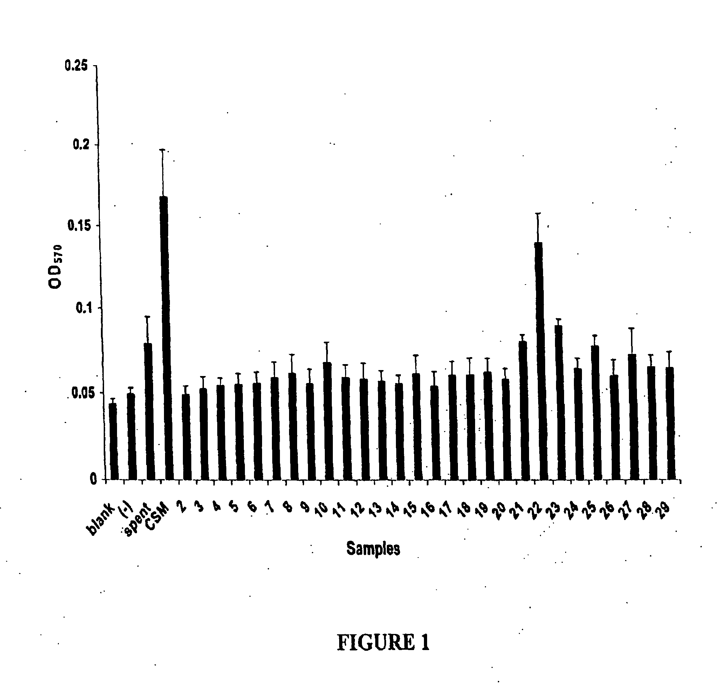

Figure 1 is a graph showing the effect of HPLC C18 reverse phase column fractionation of CSM on dispersion activity. This shows the results of microtiter plate *P. aeruginosa* 30 dispersion bioassay. Biofilms of *P. aeruginosa* were grown in microtiter plate wells and exposed for one hour to HPLC fractions of the CSM separation. Optical density values for each sample represent the density of bacteria released from biofilm during the treatment. Control wells contained EPRI (blank), *P. aeruginosa* with EPRI (-), *P. aeruginosa* with spent medium and *P. aeruginosa* with CSM. Results are the average of 8 replicates.

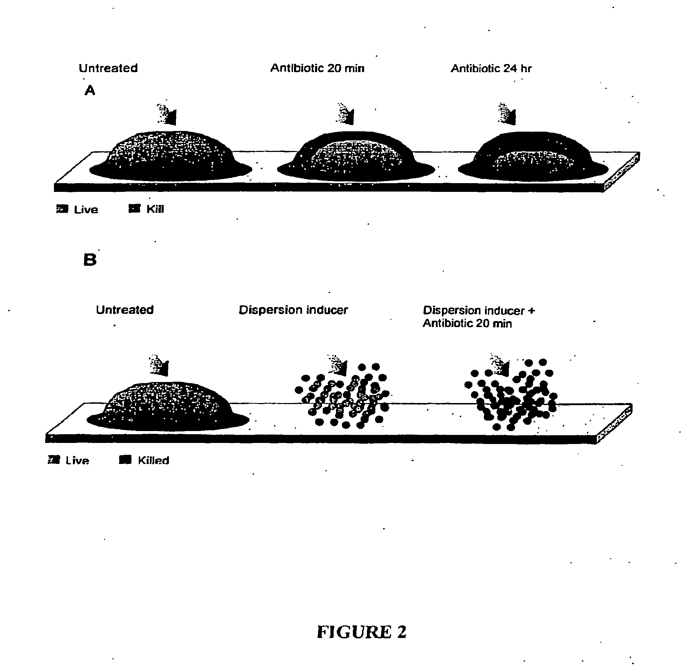

**Figures 2A-B** are schematic representations of biofilm treated with an antibiotic and/or a dispersion inducer. As shown in **Figure 2A,** following treatment, only the cells on the surface of the biofilm are killed by the antibiotic. **Figure 2B** is a schematic representation of a biofilm induced to disperse along with treatment with antibiotic. Dispersed cells are completely killed during the treatment.

**Figures 3A-C** depict the effect of addition of chloroform extracted spent culture medium (CSM) which contains a dispersion inducing compound, to mature biofilms of *Pseudomonas aeruginosa.* **Figure 3A** shows biofilm growing in continuous culture on a glass slide in a flow cell. **Figure 3B** shows the same area of biofilm 5 minutes after the addition of the dispersion inducer. **Figure 3C** shows complete dis-aggregation of biofilm following 30 minutes initial treatment with dispersion inducer.

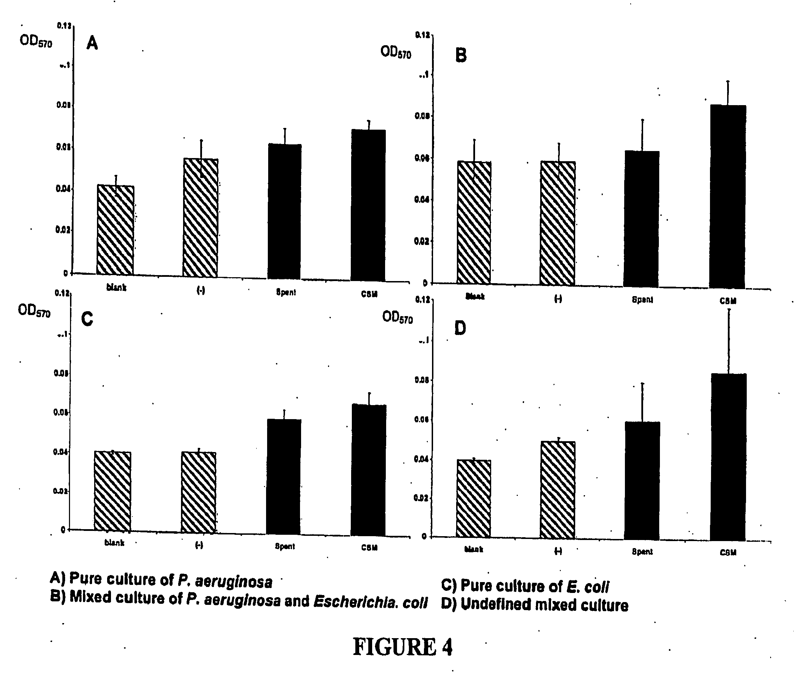

**Figures 4A-D** depict dispersion caused by chloroform extracted spent culture medium (CSM) in different bacterial cultures using a microtiter plate dispersion bioassay. **Figure 4A** is a pure culture of *P. aeruginosa.* **Figure 4B** is a mixed culture of *P. aeruginosa* and *Escherichia coli.* **Figure 4C** is a pure culture of *E. coli.* **Figure 4D** is an undefined mixed culture. Blank wells contained uninoculated EPRI; negative controls (-), contained *P. aeruginosa* with EPRI alone. Bars represent control biofilms or biofilms treated with dispersion inducer. Results are the average of 16 replicates.

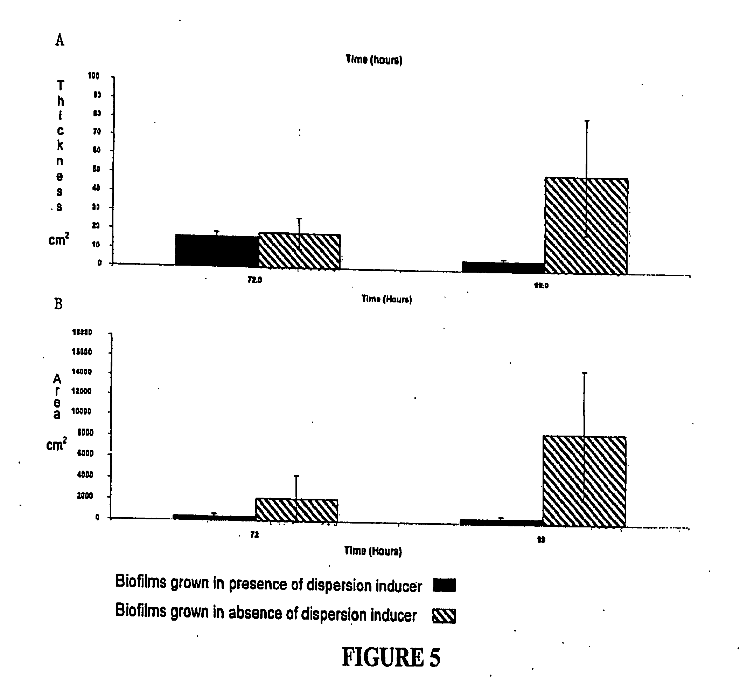

**Figures 5A-B** show biofilm development in the presence or absence of dispersion inducer. The average thickness and surface area of biofilms grown in the presence of dispersion inducer is significantly less than for untreated biofilms. Dark bars are biofilms treated with dispersion inducer during growth. Hatched bars represent biofilms grown in the absence of dispersion inducer.

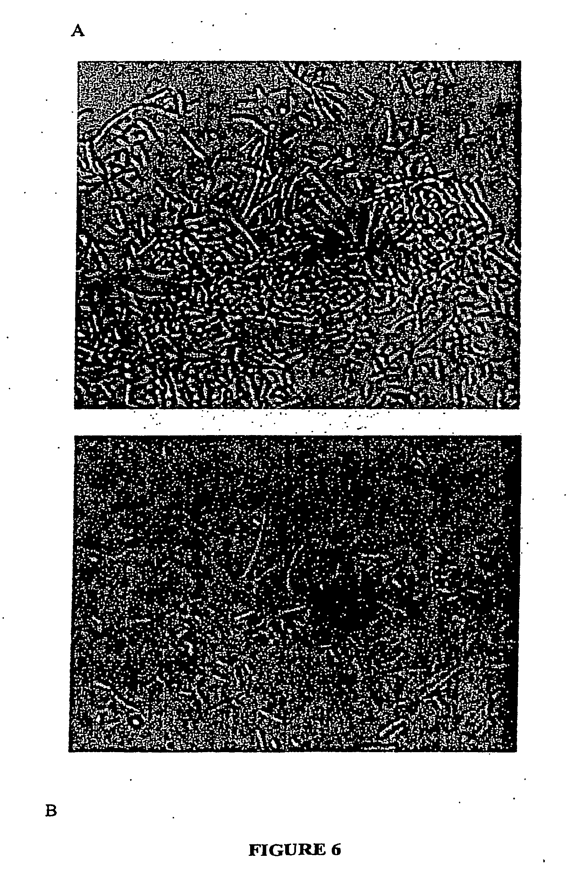

**Figures 6A-B** are phase contrast photomicrographs of biofilm 20 dispersion induced by cessation of flow. **Figure 6A** shows early biofilm development under flowing conditions, while **Figure 6B** is the same location after flow is stopped for 72 hrs.

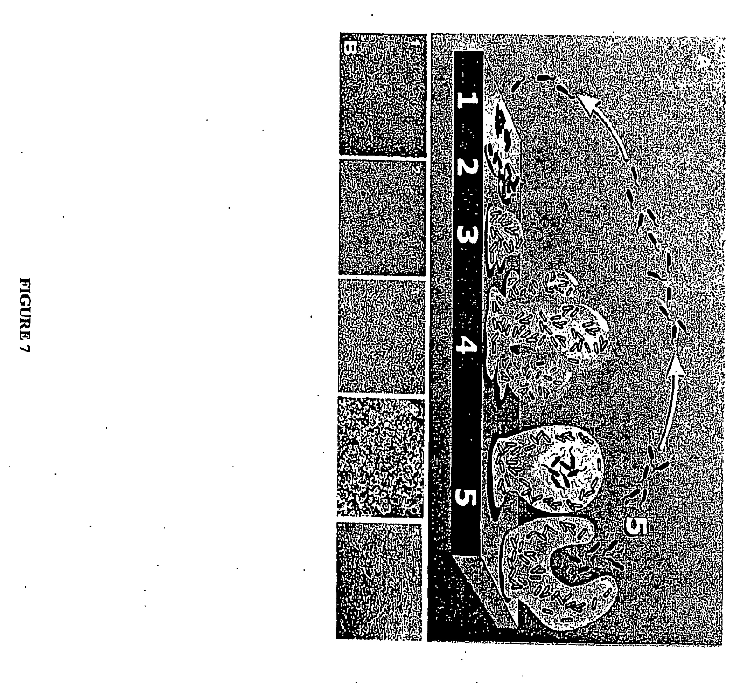

**Figure 7A** is a schematic representation of the life cycle of a biofilm. **1**, Planktonic bacteria are transported (actively and passively) to the substratum. **2**, Cell surface molecules interact with the substratum resulting in reversible surface attachment. 3, Phenotypic changes in the bacterial cell result in cell surface modifications and the production of extracellular polymeric substances, which irreversibly cement the cells to the surface. 4, Physiological changes continue with alterations in metabolism, cell-cell signaling and morphology as biofilm maturation occurs. 5, Cells release degradative enzymes to digest matrix polymer material and alter surface appendages as biofilm detachment occurs. The series of photomicrographs at the bottom of Figure 7B, show phase contrast photomicrographs of the five stages of biofilm development by *P. aeruginosa* grown in continuous culture in a flow-cell and imaged by microscopy. (Sauer et al., "Pseudomonas aeruginosa Displays Multiple Phenotypes During Development as a Biofilm," J. Bacteriol. 184:1140-1154 (2002)).

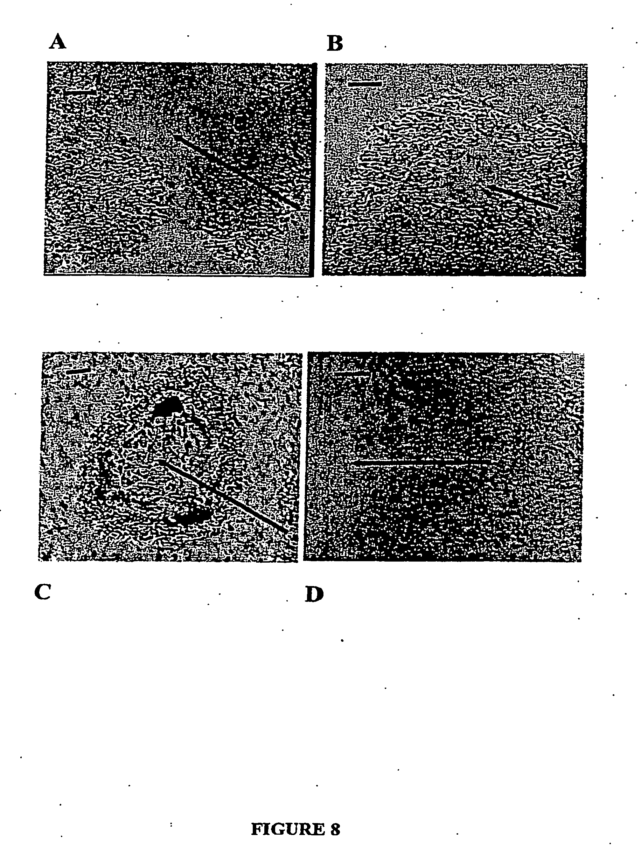

Figures 8A-D show cell clusters of *P. aeruginosa* biofilms grown in continuous culture demonstrating dispersion response. During this stage of biofilm development, bacteria become motile within cell clusters and exit into the bulk liquid through a breach in the cluster wall. Each image shows cell clusters whose interiors have been voided in this manner. Arrow indicates location of void. Bar represents 10 μm.

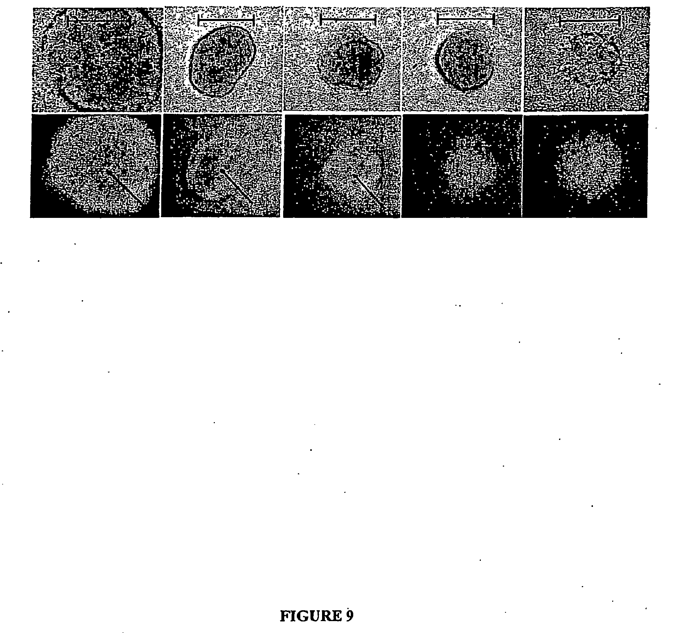

Figure 9 shows size dependence of dispersion response. Cell clusters growing in continuous culture having dimensions of greater than 40 μm diameter x 10 μm thickness show dispersion (left 3). Cell clusters below this minimum dimension remain "solid" (right 2). Top image panel, transmitted light. Bottom image panel fluorescence indicates presence of cells (*lasB*/*lacZ* reporter on chromosome). All images are same relative size. Bars represent 40 μm. Arrows indicate void areas within clusters.

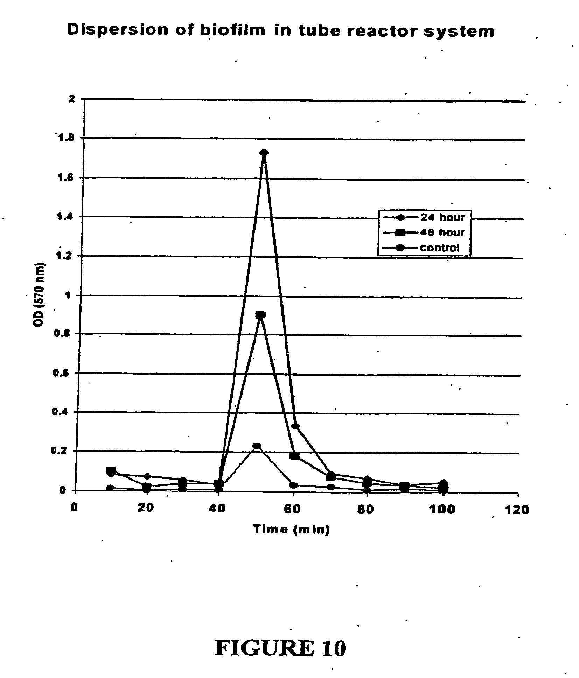

Figure 10 is a graph showing the effect of treatment of growing biofilms with spent medium. At 30 min., biofilms grown in silicone tubing for 6 days were exposed to 24 and 48 hr spent medium or control medium. Effluent was collected at the end of each tube assayed.

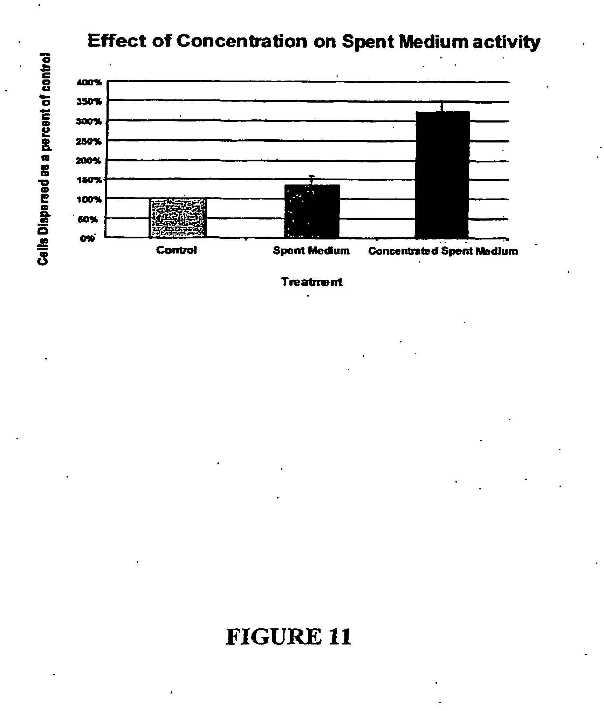

Figure 11 is a graph showing the effect of chloroform extraction on spent medium dispersion activity. Biofilms were cultured for six days in continuous culture in EPRI medium in biofilm tube reactors (Sauer et al., "Pseudomonas aeruginosa Displays Multiple Phenotypes During Development as a Biofilm," J. Bacteriol. 184:1140-1154 (2002)) and treated with spent medium (control) and chloroform extracted spent medium (CSM). Cell dispersion was determined as the optical density of culture effluent collected at the end of culture tubes. The error bar represents standard deviation for three replicate experiments.

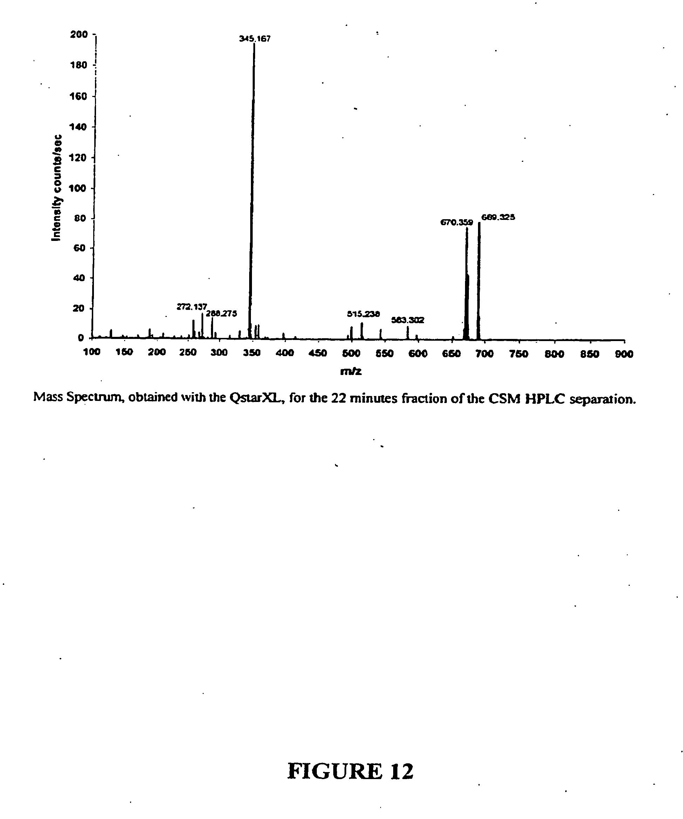

Figure 12 is a QSTAR®XL, quadrupole time-of-flight mass spectrometer mass spectrum of 22 minute HPLC fraction of CSM, showing relative intensity (counts/sec) Vs m/z (mass over charge) of peaks in sample. A Bayesian peptide reconstruction, shows the peaks at 689 mw and 345 mw to be a double charged molecule having a mass average of 688.61 mw.

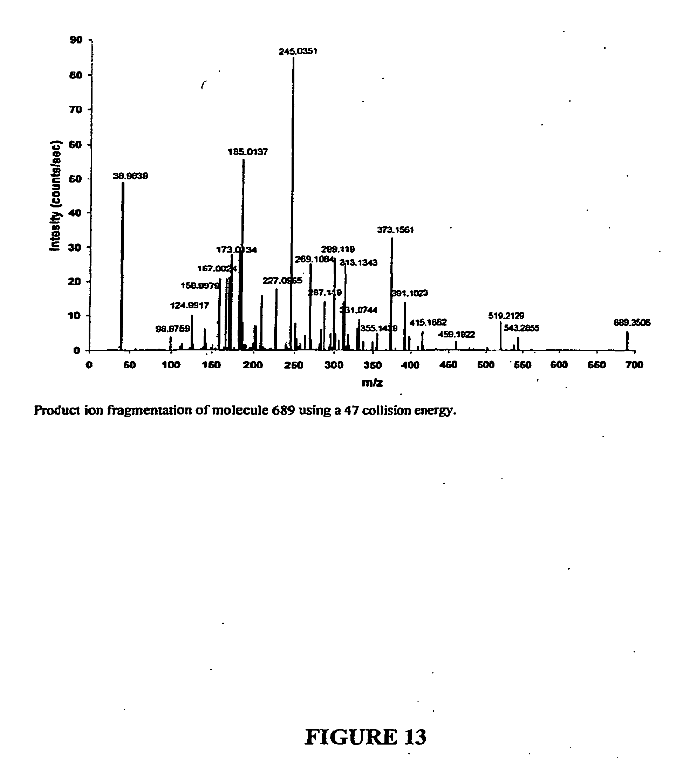

Figure 13 shows a product ion fractionation of molecule having a mass of 689, analyzed using a QSTAR®XL, quadrupole time-of-flight mass spectrometer, using a collision energy of 47.

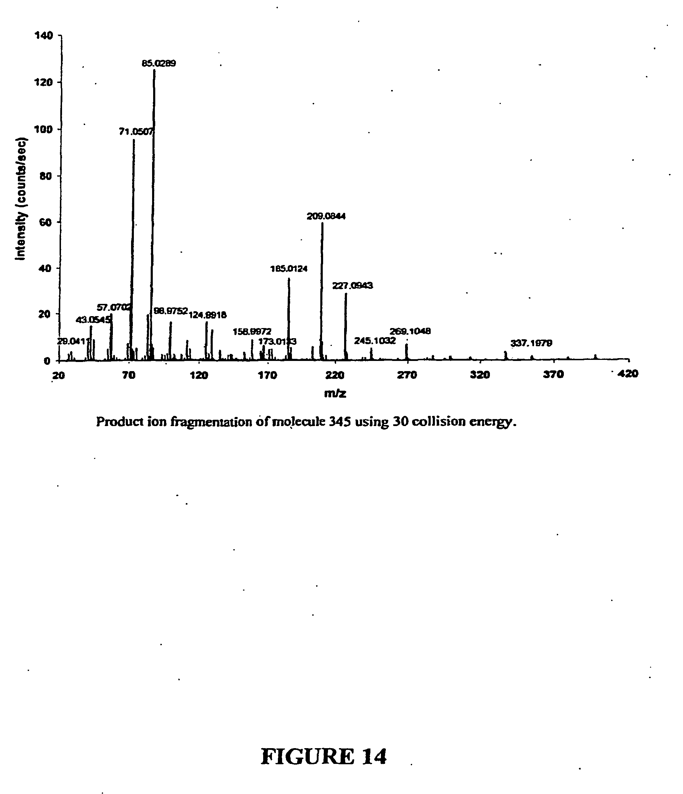

Figure 14 shows a product ion fractionation of molecule having a mass of 345, analyzed using a QSTAR®XL, quadrupole time-of-flight mass spectrometer, using a collision energy of 30.

## DETAILED DESCRIPTION OF THE INVENTION

We provide a compound which induces a physiological dispersion response in bacterial cells in a biofilm. The compound is obtained from bacteria, and preferably from *Pseudomonas aeruginosa.* We also provide a surface coated with the compound.

We provide a composition which includes the compound, and an additive component selected from the group consisting of biocides, surfactants, antibiotics, antiseptics, detergents, chelating agents, and virulence factor inhibitors. We also provide a surface coated with the composition.

We provide a method of isolating a compound which induces a physiological dispersion response in bacterial cells in a biofilm. The method involves providing a bacterial cell culture and subjecting the bacterial cell culture to a chemical extraction procedure. The chemically extracted bacterial cell culture is subjected to a high pressure liquid chromatography procedure. The method is performed under conditions effective to isolate the compound which induces a physiological dispersion response in bacterial cells in a biofilm. The compound is preferably obtained from *Pseudomonas aeruginosa* bacteria.

The present invention also relates to a method of dispersing a biofilm as set out in the claims. The method involves administering to the biofilm the compound, according to the present invention, under conditions effective to disperse the biofilm. The compound is obtained from *Pseudomonas aeruginosa.* The method of dispersing the biofilm may further include administering to the biofilm, in conjunction with administering the compound, an antimicrobial treatment. The treatment includes, but is not limited to, the administration of biocides, surfactants, antibiotics, antiseptics, detergents, chelating agents, virulence factor inhibitors, ultrasonic treatment, radiation treatment, thermal treatment, and mechanical treatment.

The present invention also relates to a method of inhibiting formation of a biofilm on a surface as set out in the claims. The method involves administering to the surface the compound according to the present invention under conditions effective to inhibit formation of a biofilm on the surface. The compound is obtained from *Pseudomonas aeruginosa.*

In one embodiment, the surface to be treated includes catheters, respirators, ventilators, stents, artificial valves, joints, pins, and other temporary or permanent devices. In another embodiment, the surface to be treated includes drains, tubs, kitchen appliances, countertops, shower curtains, grout, toilets, industrial food and beverage production facilities, and flooring. In a further embodiment, the surface to be treated is a heat exchanger surface or a filter surface. Thus, treatment provides a means for reducing the degree of biofouling of the heat exchanger or filter. In a final embodiment, the surface to be treated is a marine structure which includes boats, piers, oil platforms, water intake ports, sieves, and viewing ports. The treatment may be accomplished by applying a coating, such as paint.

The method of inhibiting formation of a biofilm on a surface may further involve administering to the surface, in conjunction with administering the compound, an antimicrobial treatment. The treatment includes, but is not limited to biocides, surfactants, antibiotics, antiseptics, detergents, chelating agents, virulence factor inhibitors, ultrasonic treatment, radiation treatment, thermal treatment, and mechanical treatment. In one embodiment, the compound and the antimicrobial treatment are administered simultaneously. In another embodiment, the compound and antimicrobial treatment are administered separately.

Alternatively, the compound is impregnated in a surface in order to inhibit formation of a biofilm on the surface. Alternatively, this objective can be achieved by administering the compound in a copolymer or a gel coating over the surface.

The present invention also relates to compositions for use in treating subjects with burns. The composition is for administering the compound, under conditions effective to treat burns in the subject. The compound is obtained from *Pseudomonas aeruginosa* as set out in the claims. A specific application of the invention provides a topical dressing for burn patients comprising dispersion inducing molecules or their natural or synthetic analogs to prevent the development of infectious biofilms or to disperse the cells of existing infectious biofilms.

The present invention further relates to compositions for use in treating and/or preventing dental plaque, dental carries, gingival disease, and oral infection in a subject. The method involves treating the oral cavity of the subject with the compound, where treating is carried out with a dentifrice, mouthwash, or dental floss containing the compound. The compound is obtained *Pseudomonas aeruginosa.*

The present invention also relates to a method of cleaning and/or disinfecting contact lenses. The method involves treating contact lenses with a cleaning and/or disinfecting solution containing the compound according to the present invention. The compound is obtained from *Pseudomonas aeruginosa.*

The present invention further relates to a composition for use in treating and/or preventing acne or other biofilm-associated skin infections on the skin of a subject. The composition is for treating the skin of the subject with the compound under conditions effective to treat and/or prevent the acne or biofilm-associated skin infections. The compound is obtained from *Pseudomonas aeruginosa* as set out in the claims. The compound may be present in an ointment, liniment, salves, shaving lotion, or aftershave.

The present invention also relates to compositions for treating and/or preventing a chronic biofilm-associated disease in a subject. The composition is for administering to the subject the compound under conditions effective to treat and/or prevent the chronic biofilm-associated disease. The compound is obtained from *Pseudomonas aeruginosa* as set out in the claims. The chronic biofilm-associated diseases to be treated and/or prevented include, but are not limited to, middle ear infections, osteomyelitis, prostatitis, cystic fibrosis, colitis, vaginitis, and urethritis. The compound may be administered in combination with antibiotics or other anti-infective treatments.

The composition for use in treating and/or preventing a chronic biofilm-associated disease in a subject may be for administering to the subject, in conjunction with administering the compound, an antimicrobial agent including, but not limited to biocides, surfactants, antibiotics, antiseptics, detergents, chelating agents, and virulence factor inhibitors.

We provide factors produced by the bacterium *Pseudomonas aeruginosa* and other bacteria capable of inducing *P. aeruginosa* and other bacteria to undergo a physiologically mediated dispersion response, resulting in the dis-aggregation of surface associated bacterial populations and communities known as biofilms. The factors that result in the disaggregation of *P. aeruginosa* biofilms, can be recovered from growing cultures of *P aeruginosa* and purified using chemical extraction methods and HPLC fractionation. The purified product can be added back to growing or mature biofilms of *P. aeruginosa* or other bacteria in pure or mixed culture and result in a dispersion response by the bacteria. These dispersed bacteria can be simultaneously or subsequently treated with anti-bacterial agents such as surfactants, biocides, or antibiotics resulting in treatment that is more effective in removing or killing biofilm bacteria than these treatments would be in the absence of the dispersion inducer.

In another application, we provide for an additive to cleaning solutions for the cleaning and disinfecting of surfaces, for example hard surfaces or woven surfaces. Examples of surfaces which may be cleaned or disinfected include toilet bowls, bath tubs, drains countertops, and kitchen appliances, carpets, laundry, surfaces in industrial settings such as food and beverage manufacturing or processing facilities, in hospital or other institutional facilities, etc.

We provide a method of disrupting growing or mature bacterial biofilms. The method involves administering a composition composed of naturally derived or synthetic biofilm auto-dispersion inducing compounds. The method is intended as a means to control or mitigate the accumulation of bacterial surface associated populations or communities and can be used independently or in combination with existing and future bacterial control strategies including, but not limited to, biocide applications, cleaning and disinfecting applications and antimicrobial chemotherapeutic agent administrations.

## EXAMPLES

The following examples are provided to illustrate embodiments of the present invention but are by no means intended to limit its scope.

## Example 1- Procedure for Extracting and Purifying Spent Medium

*Pseudomonas aeruginosa* were cultured in EPRI composed $NH_4NO_3$ (0.05 g/L), $Na_2SO_4$ (0.05 g/L), $KH_2PO_4$ (0.19g/L), $K_2HPO_4$ (0.63 g/L), Hutner Salts (1mL/L), and glucose (2.0 g/L). Four liters of EPRI were inoculated with 6 mL of an overnight culture of *P. aeruginosa* and incubated for 10 days at 30°C with agitation. Bacterial cells were harvested by centrifugation (Sorvall RC 5B plus, GSA rotor, Dupont, Ashville, NC) at 27,500 x g for 35 minutes at 4°C. Supernatant was filter-sterilized initially with a 0.45$\mu$m filter unit followed by a Millex-GP 0.22 $\mu$m filter unit (Millipore, Billerica, MA). The organic components of spent medium were extracted with chloroform (1:3.125) using a rotavapor R-3000 (Biichi Laboratories, Flawil, Switzerland) and re-suspended in 6 mL of filtered nanopure water. The final 15 product was called Chloroform extracted Spent Medium (CSM).

CSM was fractionated by High Performance Liquid Chromatography (HPLC) (System Gold HPLC, Beckman Instruments, Inc., Fullerton, CA) with a C18 Econosil reverse phase column (Alltech Associates Inc.) dimensions 250 x 4.6 mm. The column was loaded with 100 $\mu$L of CSM and eluted in an acetonitrile/water gradient (0-75%) with a flow rate of 1 mL/min for 29 minutes. Samples were collected every minute from 2 minutes onwards. The active fraction of the CSM HPLC fractionation was found to elute at 22 minutes.

A second HPLC fractionation was performed on the 22 minutes sample of CSM separation in a different acetonitrile/water gradient (40-75%) with a flow rate 25 of 1 mL/min for 35 min. From this separation the active fraction was the 32 minutes sample. The active HPLC fraction has been found to elute from a C-18 reverse phase HPLC column at a 75% acetonitrile, 25% water mixture.

The active fraction of each HPLC separation was determined by a bioassay. To enable this determination, HPLC fractions were concentrated in a Speed  Vac concentrator (Savant Instruments, Inc., Hicksville, NY) and ressuspended in 0.5 mL of filtered nanopure water.

The bioassay was performed in multiwell plates (Nalge Nunc International, Rochester, NY). Multiwell plates were etched with acetone 24 hours prior to use. An overnight culture of *P. aeruginosa* PAO1 was diluted 1:7 in EPRI, and 150 $\mu$L were inoculated into each well of the 96 multiwell plate. Cultures were grown for 7 days at 30°C (Brunswick Scientific) with EPRI being exchanged every 24 hours in the first 5 days and every 12 hours on the 6th and 7th days. On the 7th day, following 7 hours of incubation, the medium was replaced by 150 $\mu$L of EPRI (blank and negative controls), spent medium, CSM or one of the HPLC fractions obtained from the CSM or 22 minutes fractionations. Cultures were incubated 1 hour at 30°C (with shaking) after which the $OD_{570}$ was measured to indicate cell dispersion. Figure 1, shows the results of a series of bioassay experiments, performed as described above and in which individual fractions derived from HPLC separation of CSM were tested (2 min - 29 min). Optical density measurements were performed on liquid medium removed from each well and added to a clean microtiter plate to determine cell density. Cell density measurements greater than that recorded for the negative control indicate an increase in cell number in the medium due to dispersion of bacteria from the walls of each treated microtiter plate well. Blank samples were not inoculated with cell culture and were treated with 150 $\mu$L of fresh, sterile, EPRI medium; negative control samples were biofilm samples treated with 150 $\mu$L of fresh, sterile, EPRI medium; spent medium controls were treated with 150 $\mu$L of cell free spent culture medium in which *P. aeruginosa* was cultured for a period of 10 days; CSM samples were treated with 150 $\mu$L of a 1:10 diluted CSM; samples numbered 2 - 29 were treated with 2 min - 29 min fractions of CSM separated by HPLC (as described above). Results from these experiments show that the fraction of CSM having the highest dispersion activity is found in the 22 minute HPLC fraction **(Figure 1)**.

**Example 2 - Biofilm Bacteria are Resistant to Antibiotics**

**Figure 2A** illustrates schematically how biofilm bacteria are resistant to the addition of antibiotics, with similar resistance shown for biocides and other antimicrobial treatments. **Figure 2B** illustrates that if a dispersion inducer is added in addition to antibiotic, the dispersed bacteria lose their resistance and become susceptible to the antibiotic.

**Example 3 - Effect of Dispersion Inducing Compounds**

**Figure 3** shows an actual biofilm sample treated with the dispersion inducing compound according to the present invention, derived from cultures of *Pseudomonas aeruginosa.* In this experiment a once-through flow-cell was used to culture *P. aeruginosa* over a period of six days prior to testing with added CSM.

The flow cell was constructed of anodized aluminum, containing a chamber 1.0 mm by 1.4 cm by 4.0 cm capped with a glass cover slip. Sterile EPRI medium was pumped from a 2-liter vessel through silicone tubing to the flow cell using a Masterflex 8-roller-head peristaltic pump at a flow rate of 0.13 ml min$^{-1}$. Flow through the chamber was laminar, with a Reynolds number of 0.17, having a fluid residence time of 4.3 min. Medium leaving the flow cell was discharged to an effluent reservoir via silicone tubing. The entire system was closed to the outside environment but maintained in equilibrium with atmospheric pressure by a 0.2-$\mu$m-pore-size gas-permeable filter fitted to each vessel. Log-phase *P. aeruginosa* (approximately $10^8$ CFU/ml) were inoculated as a 3.0-ml slug dose through a septum 4 cm upstream from the flow cell under flowing conditions. Cells attached to the inner surface of the glass cover slip were viewed by transmitted light using an Olympus BX60 microscope and a 50x magnification ULWD MSPlan long working distance Olympus objective lens. All images were captured using a Magnafire cooled three-chip charge-coupled device (CCD) camera (Optronics Inc., Galena, Calif.) and stored as separate digital files for subsequent retrieval and analysis. Following development of a mature biofilm within the flow-cell, medium-flow was stopped and 3 mL of filtered CSM in sterile EPRI medium was added to the flow-cell. Transmitted light images of a single location within the flow-cell were taken before and during treatment with CSM. **Figure 3** shows images taken from such an experiment 1 min prior to addition of CSM, 5 min after addition of CSM, and 30 min after addition of CSM. Control samples were also run in the same manner as the test samples with the exception that CSM was not included with the 6 mL of added EPRI medium. Results from the control samples showed no change in biofilm cell numbers or biofilm architecture, with no dispersion evident.

In addition to dispersion of *P. aeruginosa* biofilms, the dispersion inducer molecule(s) have been demonstrated to be active against other species of pure culture biofilm, such as E. *coli,* and to be effective against both defined and undefined mixed culture biofilms **(Figures 4A-D)**. A biofilm microtiter plate assay was used to investigate whether CSM is able to induce the dispersion of biofilm cells from species other than *P. aeruginosa.* In performing these dispersion assays cultures of *P. aeruginosa,* cultures of *P. aeruginosa* mixed with *E. coli* K12, cultures of *E. coli* K12 alone, and cultures of undefined mixed bacteria collected from a lab air filter, were assayed using the dispersion bioassay described in Example 1, above. CSM was added to each well and allowed to interact with shaking cultures for one hour. The optical density of the bulk liquid from each well was then assayed for absorbance at 570 nm using an automated plate reader. This absorbance value represented the cells that were dispersed into the bulk liquid. Results from these experiments showed that CSM is able to induce dispersion of *P. aeruginosa* biofilms mixed with *E. coli,* biofilms composed solely of *E. coli,* and undefined mixed-culture biofilms.

Furthermore, the dispersion inducer has an inhibitory effect on the development of biofilms on uncolonized surfaces, preventing normal biofilm development Bacteria grown in the presence of the dispersion inducer fail to develop into mature biofilms, as shown in **Figures 5A-B**. In this experiment, a flow cell was used to cultivate bacteria on the surfaces of the glass substratum (described in Example 1, previously). Biofilms of *P. aeruginosa* were grown at 25°C over a period of 99 hours in the presence and absence of CSM in EPRI medium. During the course of the 25 experiment, the average biofilm thickness and total cell coverage of the bacteria on the surfaces of the flow-cell were determined by counting 20 microscope fields using a 50 x objective lens for each time point. Using the image analysis software ImagePro Plus, the total area of cells per cm$^2$ was calculated at 72 hours and 99 hours. Thickness measurements were determined by measuring the average maximum thickness of 20 random cell clusters at 72 hours and 99 hours of biofilm growth. Control samples were grown and tested in the presence of EPRI medium with no added CSM. Results from these experiments showed that the addition of CSM caused a significant reduction in the average biofilm cell cluster thickness after 99 hours growth, compared to samples not treated with CSM (**Figure 5A).** In addition, the surface area covered by the growing biofilm was shown to be significantly reduced when biofilms were grown in the presence of CSM compared to biofilms grown in EPRI medium alone (**Figure 5B).**

**Example 4 - Biofilm Bacteria Undergo a Phenotypic Switch**

During the course of normal biofilm development, biofilm bacteria undergo a phenotypic switch at the end of maturation II stage (**Figure 7**) in which their physiology changes from a predominantly biofilm form to a predominantly planktonic form. Microscopic observations of biofilms during the dispersion phase demonstrated that bacteria within cell clusters become motile (maturation stage *P. aeruginosa* are non-motile), while the bacteria around the edges of the clusters remain fixed. The region of the cell cluster within which bacteria can swim/twitch grows in volume from a (usually) central location and eventually a breach is made in the cluster wall. The bacteria are able to swim through this breach and enter the bulk liquid phase leaving behind a void within the cell cluster.

This behavior is shown in **Figure 8**, and has been observed in all experiments completed using high power microscopy to observe *P. aeruginosa* biofilms during steady state biofilm growth. In the bottom panel of **Figure 8**, typical images of cell clusters following a dispersion event are depicted (no CSM was added in these experiments). The central region of each of these images was previously observed to contain fixed (non-motile) bacteria at a density similar to that which is seen in the cluster walls in the images. Bacteria from these clusters were initially stationary. As the cell cluster developed the bacteria in the center of the clusters were observed to become agitated and eventually, motile. Finally, cells within the center of the clusters were observed to evacuate the cell cluster, leaving a void space in their place. This progression of events has been observed to be typical of the way in which *P. aeruginosa* disperse from biofilms in continuous culture. Images shown in **Figure** 8 were acquired using a flow cell to grow biofilms to maturity as described in Example 3, above. Briefly, flow-cells were inoculated with *P. aeruginosa* PAO1 and grown for 10 days in continuous culture in EPRI medium amended with 2.0 gram per liter glucose, using flow conditions described previously (Example 3, above). Previous work by applicants has shown *P. aeruginosa* to develop steady-state biofilms following a continuous culture period of 7 to 9 days. Steady state is defined by no change in effluent cell counts (CFU) resulting from detached biofilm cells; in steady state, growth of the biofilm is balanced by detachment. Individual cell clusters were examined during the course of each experiment and assigned grid coordinates, which were reexamined periodically during the course of the experiments. Cell clusters were defined as cells embedded within an exopolysaccharide matrix attached to the substratum and lacking motility; void areas within cell clusters were determined by the observation of free-swimming bacteria within a space inside a cell cluster. Images were acquired as described in Example 3, above. The cell clusters in the images shown, each display the characteristic hollow center, with motile cells visible within the cell cluster void.

Continued study of the dispersion response has revealed that cell clusters transition through episodes of growth and dispersion; the same cell cluster often enduring many such cycles. Multiple dispersion and regrowth events generally lead to the development of cell clusters with patterns analogous to growth rings which can indicate the number of times that dispersion has occurred. Often cell clusters will detach from the substratum completely during a dispersion event (Stoodley et al., "Growth and Detachment of Cell Clusters from Mature Mixed-species Biofilms," Appl. Environ. Microbiol. 67:5608-5613 (2001)). This effect is thought to be due to weakening of attachment structures at the base of the cell cluster allowing fluid sheer to detach the cluster

### Example 5 - Cell Detachment after Medium Stagnation

Figure 6 depicts a time series of phase contrast photomicrographs showing the detachment of cells after medium stagnation of 72 hours. Flow-cell were inoculated with *P. aeruginosa* PAO1 and cultured for a period of three days, according to the method described in Example 3, above. The choice of three days for culture of these biofilms was based upon the observation that under continuous flow, cell clusters within a biofilm of *P. aeruginosa* were observed to undergo spontaneous dispersion events following 9 days of incubation. After 72 days of growth under continuous flow, medium flow was stopped and images of the cell clusters were recorded every two hours for a period of 96 hours. After 72 hours of medium stagnation, the cell clusters within the flow-cell were observed to dis-aggregate, with cells entering the bulk liquid medium as planktonic bacteria (Figure 6). These experiments demonstrated that cessation of flow induced dispersion of biofilms response. In these experiments, dispersion occurred not simply within the cell clusters, but throughout all clusters in the biofilm. Only those cells which were directly attached to the substratum were not observed to swim into the bulk liquid, as illustrated in Figure 6.

### Example 6 - Dispersion is Directly Related to the Size of Cell Clusters

Repeated observation of the dispersion response has revealed regular patterns associated with the process. It has been found that dispersion is directly related to the size but not the age of a cell cluster. Observations of hundreds of cell clusters have demonstrated that under each set of medium and flow conditions cell clusters must attain a specific size in order for dispersion to take place. The size dependence of the dispersion response is illustrated in Figure 9. During these experiments, flow-cells were used to culture *P. aeruginosa* strain PAO230 in EPRI medium supplemented with 2.0 gram per liter glucose. This strain of *P. aeruginosa* contains a *lacZ* transcriptional reporter fused to the chromosomal gene *lasB,* and is responsive to activation by the *lasI* autoinducer. Activation of the reporter gene by the autoinducer occurs following the irreversible attachment stage of biofilm development, allowing detection of cells to be made in the interior of developing and mature biofilm cell clusters (Sauer et al., "Pseudomonas aeruginosa Displays Multiple Phenotypes During Development as a Biofilm," J. Bacterial. 184: 1140-1154 (2002). Activity of $\beta$ - galactosidase

was observed by microscopy following the addition of 0.04 gram per mL of methlyumbelliferyl $\beta$-D-galactopyranoside (dissolved in *N,N*-dimethylformamide) in the influent EPRI medium. Expression of the lacZ reporter was visualized by examination under long-wave UV excitation according to the method of Davies et al., "Regulation of the Alginate Biosynthesis Gene algC in Pseudomonas aeruginosa During Biofilm Development in Continuous Culture, Appl. Environ. Microbiol. 61:860-867 (1995)). Biofilms were cultured and observed as described in Example 3, above. Size measurements were taken of random cell clusters by locating the cluster nearest to a randomly selected microscope stage coordinate. Each cell cluster was measured to determine its height by focusing from the substratum through to the apex of the cluster, and its width by measurement at the base of the cell cluster using a stage micrometer. Using this technique, it was possible to determine whether a dispersion event had taken place from within a cell cluster, by examining for the presence of fluorescent cells in the interior of each measured cell cluster. Results from these experiments showed that only cell clusters having an average diameter of greater than 40 $\mu$m and a thickness of 10 $\mu$m had undergone dispersion events. Cell clusters with dimensions below these minimum values contained a "solid" core of bacteria (Figure 9). It was further observed, that the minimum detachment size for cell clusters increased as fluid flow in the biofilm flow-cell was increased. When flow was decreased, minimum size for dispersion in a cluster became less. This observation indicated that soluble factors are likely responsible for the dispersion response.

**Example 7 - Spent Medium Induces Biofilm Dispersion**

Observations that cell clusters auto-disperse from the inside, that cessation of flow causes biofilm dispersion, and that batch cultures do not promote the development of macroscopic biofilms demonstrated that factors produced by bacteria during growth are responsible for triggering biofilm dispersion. Such factor(s) are present in the liquid medium within cell clusters as well as within spent batch culture medium. Critical experiments that confirmed that components of spent medium were capable of causing significant release of bacteria from biofilms into the bulk liquid were performed. Figure 10 illustrates results from a representative experiment in which biofilms were grown in a "tube reactor" system for six days and then treated continuously for 70 minutes with cell free spent batch culture media in which *P. aeruginosa* had been grown for 24 hr or 48 hr (control was unused medium). Bacteria were removed from the spent media by centrifugation and filtration, the media were aerated, supplemented with nutrients and the pH adjusted to neutrality. Cell-free spent medium (0.6 mL) amended with 2.0 gram per liter glucose was added to test samples and sterile EPRI medium amended with 2.0 gram per liter glucose was added to control samples. The effluent from each tube was collected in 0.5 mL aliquots every 10 minutes, following treatment and assayed for optical density at 570 nanometers to determine relative amounts of dispersion. Collection of effluent samples was begun at t = 0 min and treatment was initiated at t = 30 min. Results from this experiment showed a maximum increase in the number of cells entering the effluent of the test samples (treated with 24 hour spent medium and 48 hour spent medium) 20 minutes after the addition of spent medium. This indicated that treatment with Spent Medium caused cells from the biofilm to be released into the effluent, with a maximum number of cells being released 20 minutes after exposure to Spent Medium. The small spike detectable in the control line at 50 min into the experiment was typical and probably represents a response to the physical or mechanical effects associated with a switch to new (unused) medium added during the experiment. An increase in the number of cells in the control experiments was also noted by Vats et al., "Active Detachment of STREPTOCOCCUS mutans Cells Adhered to Epon-Hydroxylapatite Surfaces Coated with Salivary Proteins In vitro," Arch. Oral Biol. 45: 305-314 (2000).

**Example 8 - Development of Chloroform Extraction Method**

Various growth and extraction procedures were tested to develop a reliable method of extracting the active fraction of spent culture medium having dispersion inducing activity. Chloroform was chosen as the extraction solvent of choice due to its compatibility with HPLC fractionation procedures, because it resulted in a narrow range of extractable organic compounds (as determined by mass spectrometry) and because it could recover bioactive amounts of the dispersion inducing agent. The method currently used for chloroform extraction of spent medium follows: Bacterial cultures of *P. aeruginosa* PAO1 were grown in 4 liters of EPRI medium (containing: sodium lactate 0.05 g/1, sodium succinate 0.05 g/1, ammonium nitrate 50.381 g/1, $KH_2PO_4$ 0.19g/l, $K_2HPO_4$ 0.63 g/1, Hutner Salts metals solution 1ml, and glucose, 2.0 g/l) in a batch culture vessel for six days at room temperature with continuous stirring. Following growth, bacteria were removed from the culture medium by centrifugation at 10,000 x g for 20 min, followed by filtration of spent medium through a 0.22 $\mu$m pore size filter. In batches, 250 ml of filtered spent medium were mixed with 80 ml of chloroform in a separatory funnel. The chloroform fraction was removed after a separation time of 10 min. The chloroform samples were then evaporated to dryness at 70°C using a rotavapor R-3000 (Biichi Laboratories, Flawil, Switzerland) and re-suspended in 6 mL of filtered nanopure water or EPRI medium. The final product, resulting from the chloroform extraction procedure is referred to here as concentrated spent medium or CSM. **Figure 11** shows the results of comparing the effect of CSM and Spent Medium on continuous culture biofilms grown in biofilm tube reactors. CSM and Spent medium were prepared as described previously from cultures of *P. aeruginosa* PAO1 grown at 22°C for 9 days in EPRI medium supplemented with 2.0 gram per Liter of glucose. Biofilms of *P. aeruginosa* PAO1 were cultured for six days at 22°C in biofilm tube reactors consisting of 32 cm silicone dioxide Masterflex size 14 tubing. At the end of six days, 6 mL of CSM, Spent Medium or Sterile EPRI medium, each supplemented with 2.0 gram per Liter glucose, was added to the tubes. The effluent from the tubes was collected and pooled for each treatment over a period of 20 minutes. Pooled sampled were assayed for optical density at 570 nm to determine relative cell numbers dispersed from each treatment. Each experiment was performed with five replicates. Results from these experiments demonstrated that chloroform extracted spent culture medium, CSM showed a greater activity in dispersing biofilms of *P. aeruginosa* compared to spent medium.

**Example 9 - Mass Spectrometry analysis of CSM HPLC fractions.**

HPLC fractions were analyzed with a QSTAR® XL, a quadrupole time-of-flight mass spectrometer (Applied Biosystems, Foster City, CA, USA) in positive electrospray ionization mode. Samples were diluted 1:2 in methanol with 0.2% formic acid and injected into the instrument source through a syringe pump. The parameters used were run using the computer program Analyst 1.1 (Applied Biosystems). Each of the peaks of interest from the resultant spectra of the active fractions (that caused dispersion) were selected for product ion analyses giving a fragmentation pattern that could enable the prediction of the structure of the molecule of interest. The spectrum obtained was plotted in intensity (counts/sec) Vs m/z (mass over charge). The product ion of each molecule was obtained with different collision energies, bigger and less charged molecules need higher collision energy for the total fragmentation of the molecule to be obtained.

Spent medium of a 10 day old *P. aeruginosa* culture was isolated and concentrated (following procedures described previously). CSM was fractionated by HPLC using a water/acetonitrile gradient (as described previously). HPLC fractions were analyzed by bioassay to determine the bio-active fractions having dispersion inducing activity. Previous results showed biofilm dispersion to occur in the positive controls (spent medium and CSM) and between 21 and 23 minutes.

CSM HPLC fractions were subsequently analyzed using a QSTAR® XL, a quadrupole time-of-flight mass spectrometer. Samples presented distinctive spectra with the bio-25 active HPLC 22 minute sample displaying the spectrum observed in **Figure 12**.

When doing Bayesian peptide reconstruct, the peaks 689 and 345 turned out to be the same molecule - 688- a double charged molecule (mass average of 688.61), with the following isotope peaks, as shown in Table 1.

**Table 1: Isotope Peak Distribution of the Molecule 688.**

| Isotope | % of | m/z | Z | Total intensity |
|---|---|---|---|---|
| | | 345.1672 | 2 | 1134.0324 |
| | | 345.6669 | 2 | 306.1008 |
| | | 346.1657 | 2 | 68.9984 |
| | | 346.6675 | 2 | 10.3312 |
| M | 79.19 | 689.3246 | 1 | 627.2474 |
| M+1 | 16.24 | 690.3219 | 1 | 131.3285 |
| M+2 | 4.57 | 691.3177 | 1 | 31.9457 |
| M+3 | 0.78 | 692.3134 | 1 | 3.6010 |

With the isotope peak M+1 the carbon content of the molecule is estimated by calculating its relative abundance

$$\frac{(\% \, M + 1 \times 100)}{\% M},$$ thus, 20 ($\pm$ 30 %) carbons could be part of this molecule.

Both molecules 345 and 689 were chosen to subsequently be analyzed by product ion fractionation **(Figures 13 and 14)**. In both fragmentation patterns **(Figures 13 and 14)**, there is an overlay of some peaks, despite them being smaller in the 679 than in the 345.

Although preferred embodiments have been depicted and described in detail herein, it will be apparent to those skilled in the relevant art that various modifications, additions, substitutions, and the like can be made. These are therefore considered to be within the scope of the invention as defined in the claims which follow.

**Claims**

1. An *in vitro* method for dispersing a biofilm or inhibiting formation of a biofilm on a surface comprising: administering to the biofilm or surface an inducer of physiologic dispersion response in bacterial cells in a biofilm, wherein the inducer is obtained by an extraction method comprising:

   providing a bacterial cell culture of *Pseudomonas aeruginosa* strain PA01 in 0.05 g/l $NH_4NO_3$, 0.05 g/l $Na_2SO_4$, 0.19 g/l $KH_2PO_4$, 0.63 g/l $K_2HPO_4$, 1 ml/l Hutner salts, and 2.0 g/l glucose;
   harvesting the *Pseudomonas aeruginosa* strain PA01 cells by centrifugation at 27,500 x g for 35 minutes at 4 °C, leaving a supernatant;
   subjecting the supernatant filtered with 0.45 $\mu$m and 0.22 $\mu$m filters to an extraction procedure with chloroform; and
   selectively isolating the inducer, from 100 $\mu$l of the extracted supernatant by a C-18 HPLC column eluted in an acetonitrile/water gradient of 0-75% over 29 minutes wherein the inducer is eluted at 22 minutes and a second C-18 reverse HPLC column eluted in an acetonitrile/water gradient of 40-75% over 35 minutes wherein the inducer is eluted at 32 minutes at a 75% acetonitrile, 25% water mixture.

2. The method of claim 1, wherein the surface is selected from the group consisting of catheters, respirators, ventilators, stents, artificial valves, joints, and pins.

3. The method of claim 1, wherein the surface is selected from the group consisting of drains, tubs, kitchen appliances, countertops, shower curtains, grout, toilets, industrial food and beverage production facilities, and flooring.

4. The method of claim 1, wherein the surface is a heat exchanger surface or a filter surface.

5. The method of claim 1, wherein the surface is a marine structure selected from the group consisting of boats, piers, oil platforms, water intake ports, sieves, and viewing ports.

6. The method of any one of the preceding claims, further comprising:

   administering to the biofilm or surface, in conjunction with said administering the inducer, an antimicrobial treatment selected from the group consisting of administration of biocides, surfactants, antimicrobials, antibiotics, antiseptics, detergents, chelating agents, virulence factor inhibitors, ultrasonic treatment, radiation treatment, thermal treatment, and mechanical treatment.

7. The method according to claim 6, wherein the inducer and the antimicrobial treatment are administered concurrently.

8. The method according to claim 6, wherein the inducer and antimicrobial treatment are administered separately.

9. The method according to any one of the preceding claims, wherein the inducer is impregnated in the surface or wherein the inducer is administered in a copolymer or a gel coating over the surface.

10. The method according to any one of claims 1or 6, wherein the surface is the surface of a contact lens.

11. A composition comprising an inducer as defined in claim 1 for use as a medicament.

12. The composition for use of claim 11 for use in dispersing a biofilm or inhibiting formation of a biofilm on a surface.

13. The composition for use of claim 11 or 12 for use in treating subjects with bums, or for use in treating and/or preventing dental plaque, dental caries, gingival disease, or oral infection in a subject, or for use in treating and/or preventing acne or other biofilm-associated skin infections on the skin of a subject, or for use in treating a chronic biofilm-associated disease selected from the group consisting of middle ear infection, osteomyelitis, prostatitis, cystic fibrosis, colitis, vaginitis, and urethritis.

14. The composition for use according to claim 11 or 12 for use in treating and/or preventing dental plaque, dental caries, gingival disease, or oral infection in a subject, wherein said treating is carried out with a dentifrice, mouthwash, or dental floss containing the composition.

**15.** The composition for use according to claim 13 or 14 wherein the composition is for administering concurrently with an antimicrobial agent selected from the group consisting of biocides, surfactants, antibiotics, antiseptics, detergents, chelating agents, and virulence factor inhibitors.

**Patentansprüche**

**1.** In-Vitro-Verfahren zum Dispergieren eines Biofilms oder Hemmen der Bildung eines Biofilms auf einer Oberfläche, das das Verabreichen eines Induktors einer physiologischen Dispersionsreaktion in Bakterienzellen in einem Biofilm an den Biofilm oder die Oberfläche umfasst, wobei der Induktor durch ein Extraktionsverfahren erhalten wird, das umfasst:

Bereitstellen einer Bakterienzellkultur des *Pseudomonas* aeruginosa-Stamms PA01 in 0,05 g/l $NH_4NO_3$, 0,05 g/l $Na_2SO_4$, 0,19 g/l $KH_2PO_4$, 0,63 g/l $K_2HPO_4$, 1 ml/l Hutner-Salzen und 2,0 g/l Glucose;
Ernten der Zellen des *Pseudomonas* aeruginosa-Stamms PA01 durch Zentrifugation mit 27.500 x g über 35 min bei 4°C, wobei ein Überstand zurückbleibt;
Durchführen eines Extraktionsverfahrens mit Chloroform an dem Überstand, der mit 0,45-pm- und 0,22-$\mu$m-Filtern filtriert wurde; und
selektives Isolieren des Induktors aus 100 $\mu$l des extrahierten Überstands durch eine C-18 HPLC-Säule, die in einem Acetonitril/Wasser-Gradienten von 0-75 % über 29 min eluiert wird, wobei der Induktor nach 22 min eluiert wird, und eine zweite C-18 Reverse-HPLC-Säule, die in einem Acetonitril/Wasser-Gradienten von 40-75 % über 35 min eluiert wird, wobei der Induktor nach 32 min mit einem 75 % Acetonitril/25 % Wasser-Gemisch eluiert wird.

**2.** Verfahren nach Anspruch 1, wobei die Oberfläche aus der Gruppe von Kathetern, Beatmungsgeräten, Ventilatoren, Stents, künstlichen Klappen, Gelenken und Nadeln ausgewählt ist.

**3.** Verfahren nach Anspruch 1, wobei die Oberfläche aus der Gruppe von Abflüssen, Wannen, Küchengeräten, Ladentischoberflächen, Duschvorhängen, Wandbewurf, Toiletten, großtechnischen Nahrungsmittel- und Getränkeproduktionsanlagen und Bodenbelag ausgewählt ist.

**4.** Verfahren nach Anspruch 1, wobei die Oberfläche eine Wärmetauscheroberfläche oder eine Filteroberfläche ist.

**5.** Verfahren nach Anspruch 1, wobei die Oberfläche eine Schiffsstruktur ist, die aus der Gruppe von Booten, Piers, Ölplattformen, Wasseraufnahmeanschlüssen, Sieben und Besichtigungsanschlüssen ausgewählt ist.

**6.** Verfahren nach einem der vorhergehenden Ansprüche, das ferner umfasst:

Verabreichen einer antimikrobiellen Behandlung, die aus der Gruppe von dem Verabreichen von Bioziden, oberflächenaktiven Mitteln, antimikrobiellen Mitteln, Antiobiotika, Antiseptika, Detergentien, Chelatbildnern, Virulenzfaktorinhibitoren, einer Ultraschallbehandlung, einer Strahlenbehandlung, einer Wärmebehandlung und einer mechanischen Behandlung ausgewählt ist, in Verbindung mit dem Verabreichen des Induktors an den Biofilm oder die Oberfläche.

**7.** Verfahren nach Anspruch 6, wobei der Induktor und die antimikrobielle Behandlung gleichzeitig verabreicht werden.

**8.** Verfahren nach Anspruch 6, wobei der Induktor und die antimikrobielle Behandlung getrennt verabreicht werden.

**9.** Verfahren nach einem der vorhergehenden Ansprüche, wobei die Oberfläche mit dem Induktor imprägniert wird oder wobei der Induktor in einem Copolymer oder Gel, die die Oberfläche bedecken, verabreicht wird.

**10.** Verfahren nach einem der Ansprüche 1 oder 6, wobei die Oberfläche die Oberfläche einer Kontaktlinse ist.

**11.** Zusammensetzung, die einen Induktor gemäß der Definition in Anspruch 1 umfasst, zur Verwendung als Medikament.

**12.** Zusammensetzung zur Verwendung nach Anspruch 11 zur Verwendung zum Dispergieren eines Biofilms oder Hemmen der Bildung eines Biofilms auf einer Oberfläche.

**13.** Zusammensetzung zur Verwendung nach Anspruch 11 oder 12 zur Verwendung bei der Behandlung von Subjekten mit Verbrennungen oder zur Verwendung bei einer Behandlung und/oder Prävention von Zahnplaque, Zahnkaries, Zahnfleischerkrankungen oder einer Mundinfektion bei einem Subjekt oder zur Verwendung bei einer Behandlung und/oder Prävention von Akne oder anderen mit einem Biofilm in Verbindung stehenden Hautinfektionen auf der Haut eines Subjekts oder zur Verwendung bei der Behandlung einer chronischen, mit einem Biofilm in Verbindung stehenden Erkrankung, die aus der Gruppe von einer Mittelohrinfektion, Osteomyelitis, Prostatitis, Muskoviszidose, Kolitis, Vaginitis und Urethritis ausgewählt ist.

**14.** Zusammensetzung zur Verwendung nach Anspruch 11 oder 12 zur Verwendung bei einer Behandlung und/oder Prävention von Zahnplaque, Zahnkaries, Zahnfleischerkrankungen oder einer Mundinfektion bei einem Subjekt, wobei das Behandeln mit einer Zahncreme, einer Mundspülung oder Zahnseide, die die Zusammensetzung enthalten, durchgeführt wird.

**15.** Zusammensetzung zur Verwendung nach Anspruch 13 oder 14, wobei die Zusammensetzung zum gleichzeitigen Verabreichen mit einem antimikrobiellen Mittel, das aus der Gruppe von Bioziden, oberflächenaktiven Mitteln, Antibiotika, Antiseptika, Detergentien, Chelatbildnern und Virulenzfaktorinhibitoren ausgewählt ist, dient.

**Revendications**

**1.** Procédé *in vitro* de dispersion d'un biofilm ou d'inhibition de la formation d'un biofilm sur une surface comprenant : l'administration au biofilm ou à la surface d'un inducteur de réponse de dispersion physiologique dans des cellules bactériennes dans un biofilm, dans lequel l'inducteur est obtenu par un procédé d'extraction comprenant :

la fourniture d'une culture cellulaire bactérienne de la souche PA01 de *Pseudomonas aeruginosa* dans du $NH_4NO_3$ à 0,05 g/L, $Na_2SO_4$ à 0,05 g/L, $KH_2PO_4$ à 0,19 g/L, $K_2HPO_4$ à 0,63 g/L, sels d'Hutner à 1 mL/L, et glucose à 2,0 g/L ;
la récolte des cellules de la souche PA01 de *Pseudomonas aeruginosa* par centrifugation à 27 500 x g pendant 35 minutes à 4 °C, laissant un surnageant ;
la soumission du surnageant filtré avec des filtres de 0,45 $\mu$m et 0,22 $\mu$m à une procédure d'extraction avec du chloroforme ; et
l'isolement sélectif de l'inducteur, à partir de 100 $\mu$L du surnageant extrait par une colonne C-18 HPLC éluée dans un gradient d'acétonitrile/eau de 0 à 75 % sur 29 minutes où l'inducteur est élué à 22 minutes et une seconde colonne HPLC inverse C-18 dans un gradient d'acétonitrile/eau de 40 à 75 % sur 35 minutes où l'inducteur est élué à 32 minutes dans un mélange d'acétonitrile à 75 %, eau à 25 %.

**2.** Procédé selon la revendication 1, dans lequel la surface est choisie dans le groupe constitué par les cathéters, les appareils respiratoires, les appareils ventilatoires, les stents, les valves artificielles, les articulations et les broches.

**3.** Procédé selon la revendication 1, dans lequel la surface est choisie dans le groupe constitué par les drains, les baignoires, les instruments de cuisine, les comptoirs, les rideaux de douche, du mastic, les toilettes, les aménagements industriels de production d'aliments et de boissons et les revêtements de sol.

**4.** Procédé selon la revendication 1, dans lequel la surface est une surface d'échangeur de chaleur ou une surface de filtre.

**5.** Procédé selon la revendication 1, dans lequel la surface est une structure marine choisie dans le groupe constitué par les bateaux, les jetées, les plateformes de pétrole, les orifices d'admission d'eau, les tamis et les hublots d'inspection.

**6.** Procédé selon l'une quelconque des revendications précédentes, comprenant en outre :

l'administration au biofilm ou à la surface, conjointement avec ladite administration de l'inducteur, d'un traitement antimicrobien choisi dans le groupe constitué par l'administration de biocides, d'agents tensioactifs, d'antimicrobiens, d'antibiotiques, d'antiseptiques, de détergents, d'agents chélatants, d'inhibiteurs de facteur de virulence, d'un traitement par ultrasons, d'un traitement par rayonnement, d'un traitement thermique et d'un traitement mécanique.

**7.** Procédé selon la revendication 6, dans lequel l'inducteur et le traitement antimicrobien sont administrés simultanément.

**8.** Procédé selon la revendication 6, dans lequel l'inducteur et le traitement antimicrobien sont administrés séparément.

**9.** Procédé selon l'une quelconque des revendications précédentes, dans lequel l'inducteur est imprégné dans la surface ou dans lequel l'inducteur est administré dans un copolymère ou un gel revêtant la surface.

**10.** Procédé selon l'une quelconque des revendications 1 ou 6, dans lequel la surface est la surface d'un verre de contact.

**11.** Composition comprenant un inducteur tel que défini dans la revendication 1 pour utilisation comme médicament.

**12.** Composition pour utilisation selon la revendication 11 pour utilisation dans la dispersion d'un biofilm ou l'inhibition de la formation d'un biofilm sur une surface.

**13.** Composition pour utilisation selon la revendication 11 ou 12 pour utilisation dans le traitement de sujets atteints de brûlures, ou pour utilisation dans le traitement et/ou la prévention de la plaque dentaire, de caries dentaires, d'une maladie gingivale ou d'une infection orale chez un sujet, ou pour utilisation dans le traitement et/ou la prévention de l'acné ou d'autres infections de la peau associées un biofilm sur la peau d'un sujet, ou pour utilisation dans le traitement d'une maladie chronique associée à un biofilm choisi dans le groupe constitué par une infection de l'oreille moyenne, une ostéomyélite, une prostatite, une fibrose kystique, une colite, une vaginite et une urétrite.

**14.** Composition pour utilisation selon la revendication 11 ou 12 pour utilisation dans le traitement et/ou la prévention de la plaque dentaire, de caries dentaires, d'une maladie gingivale ou d'une infection orale chez un sujet, dans laquelle ledit traitement est réalisé avec un dentifrice, un bain de bouche ou du fil dentaire contenant la composition.

**15.** Composition pour utilisation selon la revendication 13 ou 14 dans laquelle la composition est destinée à une administration simultanée avec un agent antimicrobien choisi dans le groupe constitué par les biocides, les agents tensioactifs, les antibiotiques, les antiseptiques, les détergents, les agents chélatants et les inhibiteurs de facteur de virulence.

FIGURE 1

Untreated          Antibiotic 20 min          Antibiotic 24 hr

A

▧ Live     ■ Kill

B

Untreated          Dispersion inducer        Dispersion inducer +
                                              Antibiotic 20 min

▧ Live     ■ Killed

# FIGURE 2

A) Before Treatment
Biofilm Cell Cluster
Biofilm Cell Cluster

B) 5 min post Treatment

C) 30 min post Treatment

FIGURE 3

A) Pure culture of *P. aeruginosa*
B) Mixed culture of *P. aeruginosa* and *Escherichia. coli*

C) Pure culture of *E. coli*
D) Undefined mixed culture

EP 1 969 128 B1

FIGURE 4

A

B

Biofilms grown in presence of dispersion inducer ▮

Biofilms grown in absence of dispersion inducer ▨

FIGURE 5

A

B

**FIGURE 6**

**FIGURE 7**

A

B

C

D

**FIGURE 8**

**FIGURE 9**

Dispersion of biofilm in tube reactor system

FIGURE 10

FIGURE 11

Mass Spectrum, obtained with the QstarXL, for the 22 minutes fraction of the CSM HPLC separation.

# FIGURE 12

Product ion fragmentation of molecule 689 using a 47 collision energy.

# FIGURE 13

Product ion fragmentation of molecule 345 using 30 collision energy.

**FIGURE 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 74875205 P

### Non-patent literature cited in the description

- **COSTERTON et al.** Bacterial Biofilms in Nature and Disease. *Annu. Rev. Microbiol.,* 1987, vol. 41, 435-464
- The Immune Response to Bacterial Biofilms. **HOIBY et al.** Microbial Biofilms. Cambridge University Press, 1995
- **LUPPENS.** Development of a Standard Test to Assess the Resistance of Staphylococcus aureus Biofilm Cells to Disinfectants. *Appl Environ Microbiol.,* 2002, vol. 68, 4194-200
- **BROWN et al.** Resistance of Bacterial Biofilms to Antibiotics: A Growth-Rate Related Effect?. *J. Antimicrob. Chemotherapy,* 1988, vol. 22, 777-783
- **ANWAR et al.** Establishment of Aging Biofilms: Possible Mechanism of Bacterial Resistance to Antimicrobial Therapy. *Antimicrob. Agents Chemother.,* 1992, vol. 36, 1347-1351
- **MAH et al.** Mechanisms of biofilm Resistance to Antimicrobial Agents. *Trends Microbiol.,* 2001, vol. 9, 34-39
- **SAUER et al.** Pseudomonas aeruginosa Displays Multiple Phenotypes During Development as a Biofilm. *J. Bacteriol.,* 2002, vol. 184, 1140-1154
- **STEWART, P.S.** Mechanisms of Antibiotic Resistance in Bacterial Biofilms. *Int. J. Med. Microbiol.,* 2002, vol. 292, 107-113
- **DONLAN et al.** Biofilms: Survival Mechanisms of Clinically Relevant Microorganisms. *Clinical Microbiol. Reviews,* 2002, vol. 15, 167-193
- **GILBERT et al.** The Physiology and Collective Recalcitrance of Microbial Biofilm Communities. *Adv. Microb. Physiol.,* 2002, vol. 46, 202-256
- **GILBERT et al.** Biofilms In vitro and In vivo: Do Singular Mechanisms Imply Cross-Resistance?. *J. Appl. Microbiol. Suppl.,* 2002, 98S-110S
- Modeling Biofilm Accumulation. **BRYERS, J.D. et al.** Physiology Models in Microbiology. 1988, vol. 2, 109-144
- **PEYTON et al.** Microbial Biofilms and Biofilm Reactors. *Bioprocess Technol.,* 1995, vol. 20, 187-231

- **ALLISON et al.** Extracellular Products as Mediators of the Formation and Detachment of Pseudomonas fluorescens Biofilms. *FEMS Microbiol. Lett.,* 1998, vol. 167, 179-184
- **LAMED et al.** Contact and Cellulolysis in Clostridium thermocellum via Extensive Surface Organelles. *Experientia,* 1986, vol. 42, 72-73
- **O'TOOLE et al.** Biofilm Formation as Microbial Development. *Ann. Rev. Microbiol.,* 2000, vol. 54, 49-79
- Regulation of Matrix Polymer in Biofilm Formation and Dispersion. **DAVIES, D.G et al.** Microbial Extracellular Polymeric Substances. Springer, 1999, 93-112
- **LEE et al.** Detachment of Streptococcus mutans Biofilm Cells by an Endogenous Enzymatic Activity. *Infect. Immun.,* 1996, vol. 64, 1035-1038
- **BOYD et al.** Role of Alginate Lyase in Cell Detachment of Pseudomonas aeruginosa. *Appl. Environ. Microbiol.,* 1995, vol. 60, 2355-2359
- **DAVIES et al.** The Involvement of Cell-to-Cell Signals in the Development of a Bacterial Biofilm. *Science,* 1998, vol. 280, 295-298
- Investigation of Quorum Sensing in Aeromonas hydrophila Biofilms Formed on Stainless Steel. **LYNCH et al.** Biofilms - The Good, the Bad and the Ugly. 1999, 209-223
- **PUCKAS et al.** A Quorum Sensing system in the Free-Living Photosynthetic Bacterium Rhodobacter sphaeroides. *J. Bacteriol.,* 1997, vol. 179, 7530-7537
- **COSTERTON et al.** Bacterial Biofilms in Nature and Disease. *Annu. Rev. Microbiol.,* 1997, vol. 41, 435-464
- **DAVIES et al.** Autodispersion in Pseudomonas aeruginosa Biofilms. Biosciences Information Service, 2003
- **STOODLEY et al.** Growth and Detachment of Cell Clusters from Mature Mixed-species Biofilms. *Appl. Environ. Microbiol.,* 2001, vol. 67, 5608-5613
- **SAUER et al.** Pseudomonas aeruginosa Displays Multiple Phenotypes During Development as a Biofilm. *J. Bacterial.,* 2002, vol. 184, 1140-1154

- **DAVIES et al.** Regulation of the Alginate Biosynthesis Gene algC in Pseudomonas aeruginosa During Biofilm Development in Continuous Culture. *Appl. Environ. Microbiol.,* 1995, vol. 61, 860-867

- **VATS et al.** Active Detachment of STREPTOCOCCUS mutans Cells Adhered to Epon-Hydroxylapatite Surfaces Coated with Salivary Proteins In vitro. *Arch. Oral Biol.,* 2000, vol. 45, 305-314